# EUROPEAN PATENT APPLICATION

(11) **EP 0 889 118 A1**
(43) Date of publication of application: **07.01.1999**
(21) Application number: 97830436.8
(22) Date of filing: 01.09.1997
(51) Int. Cl.: C12M 1/00

(54) **A bioreactor for the growth of photosynthetic microorganisms**

(30) Priority: 04.07.1997 IT PG970017
(71) Applicant: Ingredients Technology Corporation, 06059 Todi (Perugia) (IT)
(72) Inventor: The designation of the inventor has not yet been filed
(74) Representative: Gerbino, Angelo

(57) **Abstract**

Photosynthetic microorganisms are cultivated in an enclosure, and surface waves are generated, which increases the production yield of the microorganism culture.

## Description

### FIELD OF THE INVENTION

The present invention relates to bioreactors and methods for the growth of photosynthetic microorganisms.

### BACKGROUND OF THE INVENTION

Photosynthetic organisms represent a renewable source of economically important compounds such as carotenoids; phytosterols, or phycobiliproteins, phenols, flavonoids, thiols, isoprenoids and lipoic acids. Harvesting these compounds from higher plants, however, is costly and the yield is low. Photosynthetic microorganisms such as algae, on the other hand, may contain up to 20% of their dry weight as valuable components, and are thus up to 20 times more productive than higher plants as sources of these compounds.

Large scale cultivation of photosynthetic microorganisms is limited by two factors. One is the inability of light to penetrate deep into dense cultures of microorganisms, thus restricting growth to a small volume just below the surface of the liquid growth medium. Another limitation is posed by photorespiration. Photosynthetic microorganisms exposed to intense light produce oxygen during the light dependent reactions of photosynthesis which competes with CO₂ for binding to the enzyme ribulose bisphosphate carbolylase/oxygenase, and thus inhibits photosynthesis.

It is therefore the object of the present invention to provide a bioreactor for the growth of photosynthetic microorganisms in which the aforementioned limitations are substantially reduced or eliminated.

### SUMMARY OF THE INVENTION

The present invention is based upon the novel finding that generating surface waves in a liquid culture of photosynthetic microorganisms increases the obtainable cell biomass per unit of light receiving surface of the culture. While not wishing to be bound by any particular theory, it is believed that as waves pass over the surface of the culture, flows of culture medium are generated below the surface such that an individual organism moves in a circular or an elliptical path with its major axis being perpendicular to the surface. Each cell is, thus exposed to a flash of intense light when at the top of its elliptical path (near the surface) followed by exposure to dim light at the other parts of the path (deeper below the surface). It is believed that intermittent exposure to intense light optimizes the photosynthetic efficiency of the cells and further permits the oxygen produced during the light flashes to diffuse away from the cells during the episodes of dim light. Furthermore, by having a large number of cells cycling out of phase with one another, a larger fraction of the culture volume can be utilized for growth.

Accordingly, it is the object of the present invention to provide a bioreactor for the growth of photosynthetic microorganisms comprising
(a) an enclosure for containing a liquid culture of photosynthetic microorganisms;
(b) a wave generator for generating surface waves in the culture.

Light for the photosynthetic activity of the microorganisms comes from above but may also be additionally made to come from other sides or even from within as will be explained further below.

The enclosure in which the microorganisms are grown, may have a variety of shapes and forms. For example, the enclosure may have the form of a trough, it may be an annular-shaped container, it may be an open pond, etc.

The waves generated by the wave generator arc preferably a shallow-water sinusoidal harmonic wave. This type of wave propagates without change in its shape, as its velocity depends on the wave frequency (or wave length).

The wave generator may have a variety of different designs. For example, in a bioreactor of the invention having the form of a trough or pond, the wave generator may be a paddle reciprocating in a forward-rearward direction, to generate waves along an axis, preferably a longitudinal axis, of the trough or pond. Typically, the paddle is pivotly fixed at its bottom end and oscillates in a rearward-forward direction about its pivot. In the case of an enclosure which has the form of a trough or a rectangular pond, the end of the enclosure opposite the wave generator is typically provided with an inclined plane extending downward from the surface to the bottom surface of the enclosure. Such an inclined surface maintains the wave in a state of equilibrium, avoids fall of the wave and also prevents excessive interference due to the phenomena of multiple reflection which may occur in long waves. The slope depends on the length of the resulting wave as well as on the length of the enclosure.

In the case of an annular container, the wave generator may be a board paddle positioned within the container, which has dimensions and is positioned such as to fill most of the distance between opposite walls of the container with relative movement being induced between the board and the liquid culture. Such relative movement can be induced either by rotation of such board or by rotation of the container with the board being fixed.

The production yield of the culture may at times be increased by adding artificial light sources, e.g. light transmitted through optical fibers with their ends embedded within the liquid culture. This is applicable particularly in the case of an annular container: rotation of the culture container itself, may allow it to bring different portions of the cultures, consecutively, into the zone illuminated by the artificial light source.

The cells in the liquid culture do not undergo median translation but rather move in circles or more precisely, in the case of a sinusoidal harmonic wave of the shallow-water type, in ellipses. The circular motion of the cells has a radius and a period equal to that of the surface wave. As will be appreciated, the radius of the ellipse decreased exponentially with an increase in the depth of the enclosure, with the circular motion approaching closely that of a circle.

The circular motion also gives rise in fact to rotation of each cell about its axis.

When photosynthetic microorganisms are cultured in a culture without surface waves, the photosynthetic cells in upper layers of the culture are exposed to intense light, much way above saturation (Saturation is the light level which gives rise to a maximal synthetic effect; maximal photosynthetic effect for photosynthetic microorganisms in upper layers of the culture is achieved at light levels which are very much lower than the normal outdoor light levels). The circular motion of the cells gives rise to a flash effect, namely the cells go periodically through a short period, when close to the surface, where the cells are exposed to a relatively intense light, followed by a period of exposure to dimmer light, when the cells circulate through deeper layers of the culture. The flash effect of light at upper levels is sufficient to generate photosynthetic activity within the cells. The cells circulate between the surface and deeper layer of the culture and this allows in essence to utilize a larger volume of the culture, per surface area, in cultivation of photosynthetic microorganisms. In addition, the circular motion of the photosynthetic microorganisms within the culture, allows oxygen, which inhibits photosynthesis, to be diffused through a relatively larger body of water. Thus, the overall effect of generating surface waves is a very high increase in production yield of the culture.

The period of the waves is typically made so that the exposure of the cells to light in the case of sunlight, will be close and preferably not exceed the level of light saturation, which is typically about 4500 Lux. The wave period in the case of an outdoor enclosure, which may depend on exact design particulars of the enclosure and may be within the range of about 0.2-5 Hz, typically within the range of about 0.5-2 Hz, and preferably about 1 Hz.

Typically, in conventional bioreactors of photosynthetic microorganisms, the effective culture volume, for growth and cultivation of the photosynthetic microorganisms is 120 L/m², seeing that the thickness of the effective culture basin up to about 12 cm. In the case of the biorcactor of the invention, the effective volume is increased by about 5-6 times. In addition, from reasons which have not yet been elucidated, in a case of the algae *Dunaliella bardawill,* it was found that after culturing in a bioreactor of the invention, the β-carotene content in each cell is increased by about 20-33% (from about 6% to about 8%).

The enclosure may be open, or alternatively, the enclosure may have a transparent cover. A transparent cover is useful both in order to induce a greenhouse effect, giving rise to heating of the liquid culture within the enclosure by the sun, which may be important, particularly in outdoor cultivation in cold regions. Furthermore, a closed bioreactor has the advantage of preventing contamination of the culture by other types of photosynthetic microorganisms or predators which feed on the photosynthetic microorganisms

The enclosure, particularly such having the form of a trough or an annulus, may have transparent side walls, which in fact may further increase the production yield. In addition, at times the bottom wall may also be made transparent and the enclosure will then be placed above ground, optionally with providing some light irradiation from below, e.g. by diverting some sunlight, by means of mirrors, to shine on the enclosure from below.

Nutritive substances may be added to the liquid culture continuously so as to support the growing biomass of the culture. Inorganic carbon may be supplied in a gaseous farm by bubbling CO₂ into the liquid culture medium. In the case of *Dunaliella* it was found that in order to obtain a productivity of 20 g/m²/day, 37 g CO₂/m²/day is required. The bubbling of the CO₂ into the culture liquid may be regulated by a pH control apparatus maintaining the inorganic carbon of an approximately constant level.

Harvesting of the photosynthetic microorganisms may be performed as known *per se.* Typically, in the case of many microalgae, the harvesting may take place after about 4-5 days.

The present invention also provides a method for growth and cultivation of photosynthetic microorganisms, comprising:
(a) inoculating the photosynthetic microorganisms into a liquid culture medium contained in an enclosure;
(b) generating a surface wave propagating along an axis of said enclosure.

Photosynthetic microorganisms which may be grown and cultivated in accordance with the invention may be selected from a wide variety of such microorganisms. These may include numerous genera of unicellular and multicellular algae and other photosynthetic phytoplankton. Examples are; "*Spirulina platensis (Cyanophyceae), Haemetococcus pluvialis (Chlorophyceae), Chlorella sorokiniensis (Chlorophyceae), Dunaliella salina, Dunaliella barawill (Chlorophyceae), Isochrysis galbana (Haptophyceae)*. A preferred photosynthetic microorganism in accordance with the invention are the algae *Dunaliella bardawill*, cultivated for the purpose of obtaining a natural, β-carotene enriched preparation.

### BRIEF DESCRIPTION OF THE DRAWINGS

The invention will now be demonstrated by way of non-limiting examples with reference to the accompanying drawings in which:
**Fig. 1** shows a cutaway view of a bioreactor according to an embodiment of the invention, comprising a trough having a rectangular cross-sectional shape, and
**Fig. 2** shows a cutaway view of a bioreactor according to another embodiment of the invention comprising a trough having an annular shape.

### DESCRIPTION OF A SPECIFIC EMBODIMENT

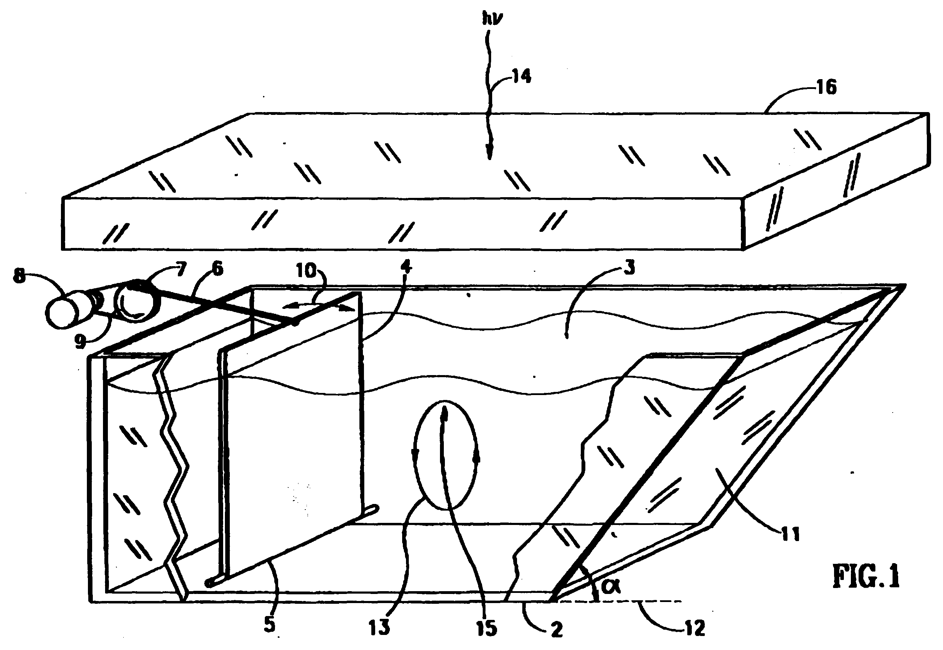

Fig. 1 shows a bioreactor **1** according to the invention in which one wall of a rectangular trough **2** has been removed to reveal the liquid culture of photosynthetic microorgarisms **3** contained within the trough. The walls of the trough are preferably transparent or translucent. Means for generating surface waves in the culture medium **3** comprises a paddle **4** near one end of the trough which is hinged at its bottom edge **5** to the bottom of the trough. A rod **6** is attached at one end to the paddle **4** while the other end of the rod is attached off-center to a disk **7**. Disk **7** can be made to rotate by means of motor **8**.

Rotation of disk **7** is a direction indicated by arrow **9** which is achieved by means of motor **8**, causes oscillation of paddle **4** around its hinged end as indicated by two-directional arrow **10**, which in turn generates surface waves in the culture **3**. The wall **11** distal to the paddle **4** is inclined so as to maximize the constructive superimposition of reflected and incident waves. The angle α of the inclined wall **11** above surface **12** depends on the frequency of the generated surface wave and on the length of the container.

An individual microorganism in the culture will undergo an elliptical oscillation in a longitudinal plane as indicated for one particular individual organism by ellipse **13**. The elliptical paths of other individual microorganisms will in general be displaced or out of phase with the elliptical path **13** shown. In addition, during the elliptical movement the microorganisms also rotate about their horizontal axis. Illumination **14** is from above and may be natural or artificial light, natural (sunlight) being preferred . The microorganisms thus experience a flash of intense light when at the top **15** of their elliptical paths **13**, otherwise they are exposed to dimmer light.

The bioreactor shown in Fig. 1 may have a depth of about 40-100 cm, typically about 50-90 cm, and preferably about 60-80 cm; and the oscillation of paddle **4** may be at a frequency within the range of about 0.2-5 Hz, typically 0.5-2 Hz and preferably at about 1 Hz.

As will be appreciated, a similar design principle to that of the bioreactor shown in Fig. 1, may also be applied to a larger enclosure, e.g. in the form of an outdoor pond. In case of a large enclosure, rather than a single wave generator, e.g. a paddle such as paddle **4** in Fig. 1, a plurality of such generators may at times be employed.

The trough shown in Fig. 1, is typically provided with a transparent cover **16** attached to the side walls of the trough in a fluid-tight manner. Nutrients may be added batch-wise or continuously through appropriate ports (not shown) and CO₂ may be continuously bubbled through liquid culture by a suitable arrangement, as known *per se* (also not shown). Excess gas, e.g. undesired oxygen produced during photosynthesis (which accumulation may inhibit further photosynthesis and thus decrease production yield) may be moved by a suitable exhaust system also known *per se* (equally not shown).

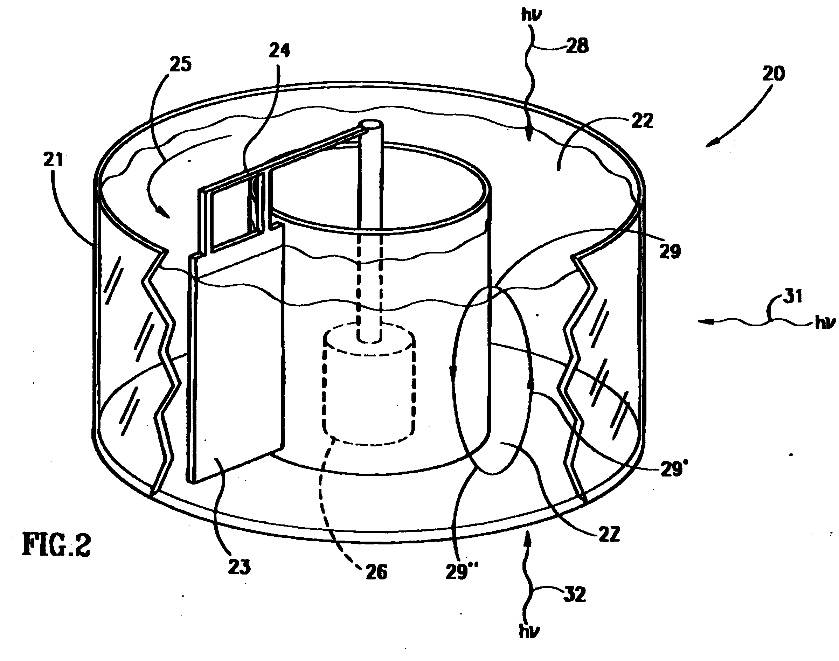

Fig. 2 shows another bioreactor **20** according to the invention comprising a trough **21** having an annular shape. One section of the outer wall of the annular trough **21** has been removed to reveal the liquid culture medium **22** contained within the trough. Means for generating surface waves in the culture medium comprises a paddle **23**. A bent rod **24** is attached at one end to paddle **23**, while the other end of the rod is attached to motor **26**.

Paddle **23** is caused to revolve continuously around the axis of the annular trough **24** as indicated by arrow **25**, by means of motor **26**, thus generating surface waves in the culture medium **22**. An individual microorganism in the culture will undergo an elliptical oscillation which in this case lies in a radial plane as indicated for one particular individual organism by ellipse **27**. In the case that the illumination **28** is from above, the organisms experience a flash of intense light when at the top **29** of their elliptical paths **27**. Where the walls of the trough are transparent light may be irradiated through either or both of the sides **31** or from the bottom **32** in which case the microorganisms experience a flash of light at several points of their elliptical path, i.e. top **29**, side **29'** and bottom **29''**. Side and bottom illumination may be achieved by incident light, by directing sunlight, e.g. by means of mirrors diverting light to the sides or bottom, or by artificial light.

It will be appreciated that rather than rotation of paddle **23**, the entire trough **21** may be rotated, with the paddle remaining stationary. Artificial light sources, e.g. optical fibers having one end embedded within culture medium **27** and the other end connected to a light source, may be added to provide auxiliary light, to increase the production yield of the microorganisms, with the culture's rotation bringing all parts of the culture into the zone illuminated with the auxiliary light.

## Claims

1. A bioreactor for the growth of photosynthetic microorganisms comprising
(a) an enclosure for containing a liquid culture of photosynthetic microorganisms;
(b) a wave generator for generating surface waves in the culture.

2. A bioreactor according to Claim 1, wherein said enclosure is a trough or pond.

3. A bioreactor according to Claim 2, wherein the wave generator is a paddle reciprocating in a rearward-backward direction to generate waves along an axis of the enclosure.

4. A bioreactor according to Claim 3, wherein the paddle is pivotally fixed at its bottom and oscillates about the pivot in a forward-rearward direction to generate said waves.

5. A bioreactor according to any one of Claims 2-4, wherein the waves are generated along a longitudinal axis of the enclosure.

6. A bioreactor according to any one of Claims 2-4, wherein the wall of the enclosure opposite the wave generator is inclined.

7. The bioreactor according to Claim 6, wherein the inclination is such so as to maintain the wave in a state of equilibrium so as to prevent fall of the wave as it approaches the opposite wall.

8. A bioreactor according to Claim 1, wherein the enclosure has the form of an annular trough.

9. A bioreactor according to Claim 8, wherein the wave generator is a paddle or board embedded within the culture medium, being in rotation movement with respect to the medium,

10. A bioreactor according to Claim 9, wherein the paddle rotates within the culture medium.

11. A bioreactor according to Claim 9, wherein the enclosure rotates and the paddle is stationary.

12. A bioreactor according to any one of Claims 1-11, the bioreactor is outdoors, and the frequency of the wave is designed so that the photosynthetic microorganisms will be exposed to light, during their passes through upper layers of the culture, which does not exceed saturation levels for photosynthesis.

13. A bioreactor according to any one of Claims 1-12, where the photosynthetic microorganisms are *Dunaliella bardawill*, cultivated for the purpose of obtaining a natural, β-carotene enriched preparation therefrom.

14. A method for growth and cultivation of photasynthetic microorganisms, comprising;
(a) inoculating the photosynthetic microorganisms into a liquid culture medium contained in an enclosure;
(b) generating a surface wave propagating along an axis of said enclosure.

15. A method according to Claim 1, wherein generated waves are shallow-water sinusoidal harmonic waves,

16. A method according to Claim 14 or 15, wherein the synthetic microorganisms are *Dunaliella bardawill*, which are cultivated for obtaining a natural, β-carotene enriched preparation therefrom.
